(19) 

**Europäisches Patentamt**

**European Patent Office**

**Office européen des brevets**

(11)  **EP 4 717 177 A1**

(12) **EUROPEAN PATENT APPLICATION**

(43) Date of publication:
01.04.2026   Bulletin 2026/14

(21) Application number: 25200437.9

(22) Date of filing: 05.09.2025

(51) International Patent Classification (IPC):
*A61B 6/00* *(2024.01)*    *A61B 6/03* *(2006.01)*
*A61B 6/04* *(2006.01)*    *A61B 5/055* *(2006.01)*

(52) Cooperative Patent Classification (CPC):
**A61B 6/0407; A61B 5/055;** A61B 6/032;
A61B 6/542; A61B 6/547

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:   30.09.2024   US 202418902101

(71) Applicant: GE Precision Healthcare LLC
Waukesha, WI 53188 (US)

(72) Inventors:
• **LEWIS, Chelsey Amanda**
**Waukesha, 53188-1696 (US)**
• **DELONG SAMALIK, Michelle Marie Severino**
**Waukesha, 53188-1696 (US)**
• **MAULE, Joseph Daniel**
**Waukesha, 53188-1696 (US)**
• **MAULE, Samuel Edward**
**Waukesha, 53188-1696 (US)**

(74) Representative: **AWA Sweden AB**
**Box 45086**
**104 30 Stockholm (SE)**

(54)   **DETERMINING A WEIGHT OF A SUBJECT ON A TABLE OF A MEDICAL IMAGING SYSTEM**

(57)    A medical imaging system includes a gantry including a bore extending through the gantry. The medical imaging system further includes a table configured to support a subject moving through the bore. The medical imaging system further includes at least one sensor disposed on the table and configured to sense a total weight of a load on the table at two or more different measurement points. The medical imaging system further includes camera configured to generate an image of the table including the subject. The medical imaging system further includes a processor configured to determine a weight of the subject on the table based on at least one signal from the at least one sensor and a second signal from the camera.

FIGURE 1

**Description**

FIELD

The following generally relates to medical imaging systems and more particularly to determining a weight of a subject on a table of a medical imaging system, and is described with particular application to Computed Tomography (CT); however, the following is also amenable to other medical imaging modalities, including, but not limited to, one or more of a Magnetic Resonance (MR) imaging system, an X-ray imaging system, a Positron Emission Tomography (PET) imaging system, and a Single Photon Emission Computed Tomography (SPECT) imaging system.

BACKGROUND

A Computed Tomography (CT) imaging system includes a gantry and a rotating frame rotatably supported by a bearing in the gantry. The gantry includes a bore, which serves as an examination region. The rotating frame includes an annular ring and is configured to rotate around the examination region along an axis of rotation about a center of rotation (e.g., a center of the bore). The rotating frame carries components such as an X-ray source and an X-ray radiation sensitive detector array, which are disposed along arcs of the ring, opposite each other across the bore. A table or subject support surface is configured to support a subject as the subject is moved through the bore.

In operation, the table loads the subject into the bore for scanning. During (pre-, axial, helical, etc.) scanning, the table supports the subject in the bore. For scanning, the rotating frame rotates around the subject, the X-ray source emits X-ray radiation that traverses the subject in the bore, and the X-ray radiation sensitive detector array detects X-ray radiation traversing the subject in the bore that impinges thereon. After scanning, the table moves the subject out of the bore.

The detector array (e.g., the X-ray radiation sensitive detector array) generates projection data (line integrals) indicative of the detected X-ray radiation. A reconstructor reconstructs the projection data and generates volumetric image data. Voxels of the reconstructed volumetric image data are displayed as a two-dimensional (2-D) CT image and/or a three-dimensional (3-D) CT image using gray scale values corresponding to a relative radiodensity. The gray scale values reflect the attenuation characteristics of the scanned subject and/or object and generally show structure such as anatomical structures within the scanned subject and/or physical structure within the scanned object. Additionally, the CT images may include colorized portions or overlays.

Prior to scanning a subject, the clinician administering the scan sets up the scan. For example, through application software or the like, the clinician enters patient identification information, selects a scan protocol, enters and/or selects scan parameters such as X-ray tube electrical potential in peak kilovolt (kVp), X-ray tube current exposure time in milliampere-seconds (mAs), etc. Certain scan protocols (e.g., pediatric, infant, etc.) and scan parameters (e.g., kVp, mAs, etc.) are selected based on a weight of the subject being scanned. For example, the kVp affects penetration power and thus contrast enhancement, and the mAs affects the number of X-rays and thus image noise and radiation dose. That is, there is correlation between subject weight and scan protocols and parameters that affects image quality and radiation dose.

With some imaging systems, the table is configured to measure a weight of a load (e.g., the subject plus any objects) carried by the table. One MR imaging system includes a MR coil detection unit on the table that receives MR coil identification information electrically through plug-type contacts from an MR coil being used for the scan or a data exchange with the control unit for the system. Another MR imaging system receives MR coil or padding identification and/or weight information electrically through a plug connection with a MR coil being used for the scan or manually via an entry by a user. With these MR systems, the weight of the coil being used and/or the padding is known and subtracted from the measured load to estimate the weight of the subject.

However, objects that have been on a table with a subject are not all in electrical communication with the imaging system (or are even capable to electronically provide information), nor are all of their weights known such that they can be entered manually by a user of the system. For instance, a subject transported to a hospital emergency room via an ambulance service might be connected to a portable electrocardiograph (ECG) device through lead wires and electrodes, which need to remain connected because the person is having chest pain, possibly from a myocardial infarction, and require a CT scan for diagnosis. ECG devices can vary in weight from manufacturer to manufacturer, and even between models of a same manufacturer. Other such objects include a backboard, a neck collar, a brace, a blanket, etc.

In such an instance, it likely is not feasible to weigh a trauma subject standing up with a standard scale, and the weight measurement by the table would be the weight of the load, which includes an aggregate weight of the weight of the subject and the weight of the ECG device and supporting components. Unfortunately, selecting a scan protocol and/or scan parameters based on an incorrect patient weight might result in over-exposing the patient to X-ray radiation (and X-ray radiation is ionizing radiation, which can damage and/or kill cells) or under-exposing the patient, leading to poorer image quality that might not be diagnostic image quality, and which might result in a rescan and additional X-ray radiation exposure to the patient.

In view of at least the foregoing, there is an unresolved need for an improved approach for determining a weight of a subject on a table of a medical imaging system.

SUMMARY

Aspects described herein address the above-referenced problems and others. This summary introduces concepts that are described in more detail in the detailed description. It should not be used to identify essential features of the claimed subject matter, nor to limit the scope of the claimed subject matter.

In one aspect, a medical imaging includes a gantry including a bore extending through the gantry. The medical imaging system further includes a table configured to support a subject moving through the bore. The medical imaging system further includes at least one sensor disposed on the table and configured to sense a total weight of a load on the table at two or more different measurement points. The medical imaging system further includes camera configured to generate an image of the table including the subject. The medical imaging system further includes a processor configured to determine a weight of the subject on the table based on at least one signal from the at least one sensor and a second signal from the camera.

In another aspect, a computer-implemented method includes receiving at least one signal from at least one sensor disposed on a table of a medical imaging system, wherein the at least one signal includes measurements from two or more different measurement points of the table. The computer-implemented method further includes receiving a second signal from an optical sensor configured to generate an image of the table. The computer-implemented method further includes determining a weight of a subject on the table based on the at least one signal and the second signal.

In another aspect, a computer readable medium is encoded with computer executable instructions. The computer executable instructions, when executed by a processor, cause the processor to receive at least one signal from at least one sensor disposed on a table of a medical imaging system, wherein the at least one signal includes measurements from two or more different measurement points of the table, receive a second signal from an optical sensor configured to generate an image of the table, and determine a weight of a subject on the table based on the at least one signal and the second signal.

Those skilled in the art will recognize still other aspects of the present application upon reading and understanding the attached description.

BRIEF DESCRIPTION OF THE DRAWINGS

The application is illustrated by way of example and not limited by the figures of the accompanying drawings in which like references indicate similar elements.

FIGURE 1 schematically illustrates a non-limiting example of an imaging system configured for Computed Tomography (CT) imaging and including at least one sensor on a table, a camera, and a subject support module, in accordance with an embodiment(s) herein.

FIGURE 2 schematically illustrates a side view of an example of the table that includes multiple sensors disposed at a first set of locations for measuring at two or more measuring points, in accordance with an embodiment(s) herein.

FIGURE 3 schematically illustrates a side view of an example subject of the stable that includes multiple sensors disposed at a different set of locations for measuring at two or more measuring points, in accordance with an embodiment(s) herein.

FIGURE 4 schematically illustrates a side view of an example subject of the table that a single sensor for measuring at two or more measuring points, in accordance with an embodiment(s) herein.

FIGURE 5 schematically illustrates an example of the subject support module configured to determine a weight of a subject on the table of the imaging system, in accordance with an embodiment(s) herein.

FIGURE 6 schematically illustrates another example of the subject support module configured to determine the weight of the subject on the table of the imaging system, in accordance with an embodiment(s) herein.

FIGURE 7 schematically illustrates yet another example of the subject support module configured to determine the weight of the subject on the table of the imaging system, in accordance with an embodiment(s) herein.

FIGURE 8 illustrates an example graphical presentation that indicates a state of a weight carried by the table, in accordance with an embodiment(s) herein.

FIGURE 9A illustrates another example graphical presentation that indicates a state of a weight carried by the table, in accordance with an embodiment(s) herein.

FIGURE 9B illustrates yet another example graphical presentation that indicates a state of a weight carried by the table, in accordance with an embodiment(s) herein.

FIGURE 10 illustrates a non-limiting example of a flow chart for a computer-implemented method for determining a weight of a subject on the table, in accordance with an embodiment(s) herein.

FIGURE 11 illustrates a non-limiting example of a flow chart for a computer-implemented method for determining whether a weight of a subject on the table exceeds a weight limit of the table, in accordance with an embodiment(s) herein.

FIGURE 12 illustrates a non-limiting example of a flow chart for a computer-implemented method for determining subject movement on the table, in accordance with an embodiment(s) herein.

FIGURE 13 illustrates a non-limiting example of a flow chart for a computer-implemented method for determining subject self-unloading from the table, in accordance with an embodiment(s) herein.

FIGURE 14 illustrates a non-limiting example of a flow chart for a computer-implemented method for determining subject self-loading on the table, in accordance with an embodiment(s) herein.

DETAILED DESCRIPTION

Embodiments of the present disclosure will now be described, by way of example, with reference to the figures, in which a system, a method and/or a set of instructions on a computer readable medium determine a weight of a subject on a table or subject support of a medical imaging system based on force and/or displacement sensor measurements at two or more different measurement points on the table and camera based identification of an object on the table. This includes instances in which not only is the subject on the table, but concurrently therewith, at least one object is on the table, and the object has an unknown weight that affects scan protocol or parameter selection and does not electrically communicate information indicative of its weight to the imaging system.

As briefly discussed above, some existing imaging systems are configured to measure a load (subject weight plus object weight) on a table, the weights of many objects commonly accompanying a patient on a table are not readily available (unknown and/or not determined from information electrically communicated to the system) such that the weights of the objects cannot readily be considered to determine the weight of the subject, there is correlation between patient weight and scan protocols and/or scan parameters that affects image quality and radiation dose, and selecting a scan protocol and/or scan parameters based on an incorrect patient weight might result in patient over-exposure to X-ray radiation and/or image quality that is less than diagnostic image quality.

As described in greater detail below, in one instance a weight of the load (the subject plus and an object) on the table is determined based on the measurements, a weight of object on the table is determined based on an analysis of a signal from the camera, and the weight of the subject is determined based on the weight of the load and the weight of the object, e.g., by subtracting the weight of the object from the weight of the load. For example, in one instance a classifier is configured to identify and classify the object on the table based on the signal from the camera, and the estimated weight of object is obtained from a mapping of objects to object weights. In one instance, determining the weight of the subject as such mitigates selecting scan parameters based on an incorrect subject weight and the above noted shortcoming therewith.

The determined subject weight can be utilized to identify whether the subject satisfies a weight limit of the table. In this instance, a notification (e.g., a warning, a message, etc.) can be presented (e.g., visual, auditory, haptic, etc.), certain functionality (e.g., X-ray production, X-ray transmission, scanning, etc.) can be disabled, prevented, and/or inhibited, etc. This can mitigate weight overload damage to the table. The determined subject weight (e.g., a change therein) can be utilized to determine that the subject is moving or is no longer on the table. Likewise, a notification can be presented, certain functionality can be disabled, prevented, and/or inhibited, etc. This can mitigate image artifact, subject injury, etc. The determined subject weight can be utilized to notify service personnel of a current state of the table, e.g., whether nothing, a service accessory, or an item (including a subject) is on the table. This can mitigate inadvertent X-ray exposure to a subject, repeating a service procedure, etc.

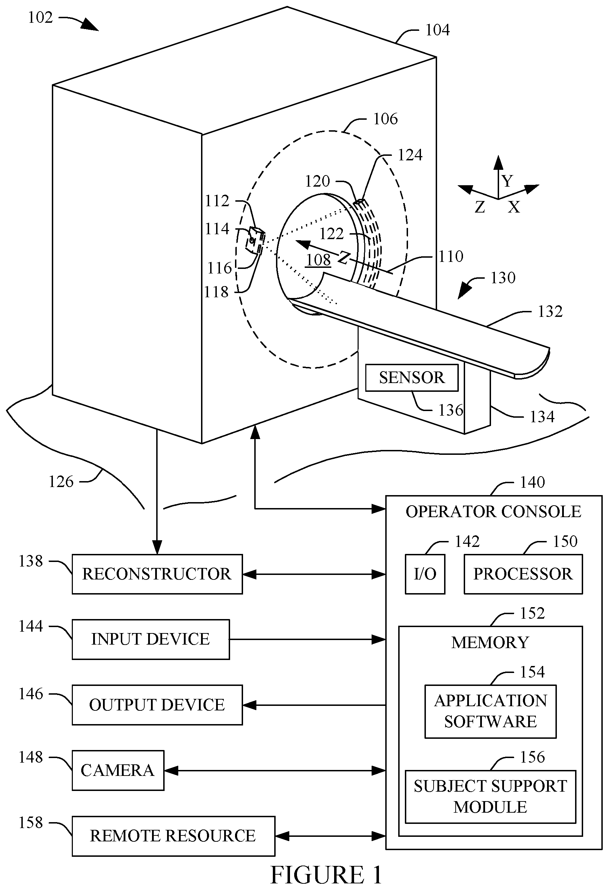

Initially referring to FIGURE 1, a non-limiting example of an imaging system 102 is schematically illustrated. In this example, the imaging system 102 is configured for Computed Tomography (CT) imaging. In another instance, the imaging system 102 is additionally or alternatively configured to include another imaging modality such as a Magnetic Resonance (MR) imaging system, an X-ray imaging system, a Positron Emission Tomography (PET) imaging system, a Single Photon Emission Computed Tomography (SPECT) imaging system, and/or other imaging system. For clarity and sake of brevity, the below discussion describes the imaging system 102 configured for CT imaging.

The imaging system 102 includes a gantry 104. In some instances, the gantry 104 is configured to tilt. The imaging system 102 further includes a rotating frame 106. The rotating frame 106 is rotatably supported in the gantry 104, e.g., via a bearing (e.g., a slip ring) or the like, and is configured to rotate around a bore 108 or examination region about a rotational or z-axis 110, which extends through a center of rotation (e.g., a center of the bore 108 (i.e., an isocenter)). A gantry controller (not visible) is configured to control rotation of the rotating frame 106 and, if configured to tilt, tilting of the gantry 104.

An X-ray source assembly 112 is supported by the rotating frame 106 and rotates in coordination with the rotating frame 106. The X-ray source assembly 112 includes an X-ray source 114 such as an X-ray tube. The X-ray source 114 is configured to emit X-ray radiation having an energy at least in the X-ray diagnostic range (e.g., 20 keV to 150 keV). The X-ray assembly 112 may further include or is coupled to a filter 116 that characterizes an X-ray radiation dose profile and/or a collimator 118 that shapes the X-ray radiation to form a generally (fan, wedge, cone, etc.) shaped beam that traverses the bore 108. An X-ray controller (not visible) is configured to control components of the X-ray source assembly 112 such as X-ray radiation emission of the X-ray source 114, the collimator 118, etc.

An X-ray radiation sensitive detector array 120 includes a one-dimensional (1-D) or two-dimensional (2-D) array of rows of X-ray radiation sensitive detector elements 122 and is supported by the rotating frame 106 along an arc opposite the X-ray source 114, across the bore 108. Each of the X-ray radiation sensitive detector elements 122 is in electrical communication with a Data Acquisition System (DAS) 124. The X-ray radiation sensitive detector elements 122 include an indirect conversion detector such as a scintillator / photodiode detector and/or a direct conversion detector such as a Cadmium Telluride (CdTe), a Cadmium Zinc Telluride (CZT), etc. detector. A DAS controller (not visible) controls the X-ray radiation sensitive detector array 120.

A table 130 includes a cradle 132 moveably coupled to a frame/base 134. In one instance, the cradle 132 is slidably coupled to the frame/base 134 via a bearing or the like, and a drive system (not visible) including a motor, a lead screw, and a nut (or other drive system) translates the cradle 132 along the frame/base 134 into and out of the bore 108 for horizontal motion, and the frame/base 134 includes a drive system (not visible) including a lift mechanism (e.g., scissor lift, hydraulics, etc.) for vertical motion. In another example, the table 130 is configured for diagonal (concurrent horizontal and vertical) motion. The cradle 132 is configured to support a subject (or an object) in the bore 108 for loading, scanning, and/or unloading the subject or object. A table controller (not visible) controls the drive system.

The table 130 further includes at least one sensor 136 configured to measure at two or more measurement points of the table 130. Examples of suitable sensors include sensors that sense information (e.g., force, displacement, etc.) that can be utilized to determine at least a weight and/or a position of a load (e.g., a subject and one or more objects) on the table 130, such as a strain gauge, a displacement sensor, a pressure sensor, a load cell, and/or the like. By way of non-limiting example, in one instance a strain gauge is configured to measure strain ($\varepsilon$, a change in length divided by the original length) of an applied load (i.e., force) through a change in an electrical resistance of the strain gauge, which can be measured. The output signal of each strain gauge is a signal indicative of the change in the electrical resistance and hence the strain. In one instance, the table 130 includes one or more signal conditioning components configured to amplify, filter, digitize, etc. the signal from the at least one sensor 136.

For a helical scan, the rotating frame 106 rotates in coordination with the cradle 132 moving along the Z-axis 110, and active X-ray detector elements 122 of the X-ray radiation sensitive detector array 120 detect X-ray radiation over consecutive arc segments (integration periods) each revolution and generate respective signals. For an axial (step and shoot) scan, the cradle 132 is positioned at a static position for each integration period and moves between integration periods. For each arc segment, the DAS 124 processes each signal and generates projection data.

A reconstructor 138 reconstructs the projection data and generates volumetric (3-D) image data for a helical scan and/or individual axial (2-D) image for an axial step and shoot scan (which can be used in combination to generate volumetric image data). The volumetric image data and/or 2-D slices thereof, and/or the individual axial images can be visually presented, filmed, etc. Examples of suitable reconstruction algorithms include filtered back projection (FBP), advanced statistical iterative reconstruction (ASIR), conjugate gradient (CG), maximum likelihood expectation maximization (MLEM), model-based iterative reconstruction (MBIR), and/or other reconstruction algorithm.

A computing system 140 serves as an "operator console" of the imaging system 102. The computing system 140 may include a computer, a workstation, server, etc. The computing system 140 includes input/output (I/O) 142. An input device 144 includes a keyboard, mouse, touchscreen, microphone, etc. The input device 144 is in electrical communication with the computing system 140 through the I/O 142 and/or otherwise. An output device 146 includes a human readable device such as a display monitor or the like. The output device 146 is in electrical communication with the computing system 140 through the I/O 142 and/or otherwise.

At least one optical sensor such as camera 148 is configured to capture an image inside of an examination room in which the imaging system 102 is installed. As utilized herein, the term "image" encompasses a single image, a collection of single images, and video. In one instance, this includes capturing an image of the table 130 such as any subject and/or object on the cradle 132 of the table 130. In one instance, the camera 148 is integrated in the imaging system 102, e.g., inside and/or outside of a cover of the gantry 104. Additionally, or alternatively, the camera 148 is mounted to a ceiling and/or a wall of the examination room. Additionally, or alternatively, the camera 148 is carried by stationary and/or mobile device such as a stand in the examination room. The at least one camera 148 can be configured to capture two-dimensional (2-D) and/or three-dimensional (3-D), color and/or black and white, electromagnetic radiation in the visible light and/or infrared bands, etc. The at least one camera 148 is in electrical communication with the computing system 140 through the I/O 142 and/or otherwise.

The computing system 140 further includes at least one processor 150 such as a microprocessor (μP), a central processing unit (CPU), graphics processing unit (GPU), etc., and a computer readable medium 152 ("MEMORY"), which includes non-transitory medium and excludes transitory medium (signals, carrier waves, and the like). The computer readable medium/memory 152 at least includes application software 154 and a subject support module 156. The application software 154 is configured to provide a user interface that allows a user to select a scan protocol, entry and/or select scan parameters (e.g., kVp, mAs, etc.) of the scan protocol, initiate scanning, stop scanning, etc. The user interface is also configured to display notifications such as notifications associated with the table 130 such as a weight of subject, a weight overload on the table 130, whether anything is on the table 130, subject motion or removal from the table 130, etc.

As described in greater detail below, in one instance the subject support module 156 receives, as input, signals from the at least one sensor 136 and a signal from the camera 148, processes the signals, and determines at least a weight of a subject on the table 130. This includes instances in which not only is the subject on the table 130, but concurrently therewith, at least one object is on the table 130, and the object has an unknown weight that affects scan protocol or parameter selection and does not electrically communicate information indicative of its weight to the imaging system. In one instance, determining the weight of the subject as such provides an accurate weight that can be utilized to select scan protocols and/or parameters, mitigating issues associated with incorrect weight estimates.

In other aspects, the determined subject weight can be utilized to identify whether the subject satisfies a weight limit of the table, determine that the subject is moving, determine that the subject is no longer on the table 130, notify personnel of a current state of the table 13-, etc. Such functionality can mitigate weight overload damage to the table 130, image artifact, subject injury, inadvertent X-ray exposure to a subject, repeating a service procedure, etc. In one or more of these instances, at least a notification can be presented to the subject and/or use of the operator console 140 indicating the weight, the overload, the movement, the removal of the subject, and/or whether nothing, an item intentionally placed on the table and/or an item that should not be on the table 130 is on the table 130. Additionally, or alternatively, for one or more of these instances, the certain functionality (e.g., X-rays on, etc.) can be disabled, prevented, and/or inhibited.

A remote resource 158 includes one or more of a server, a workstation, a Radiology Information System (RIS), a Hospital Information System (HIS), an Electronic Medical Record (EMR), a Picture Archiving and Communications System (PACS), one or more other CT scanners, cloud processing resources (which includes shared remote data storage and/or computing power, including processing resources distributed over multiple locations / data centers), etc. The remote resource 158 is in electrical communication with the computing system 140 through the I/O 142 and/or otherwise. The imaging system 102 and the remote resource 158 are configured to communicate with each other via Digital Imaging and Communications in Medicine (DICOM), Health Level Seven (HL7), etc. For example, the determined weight of a subject can be transmitted to the EMR for the subject via HL7.

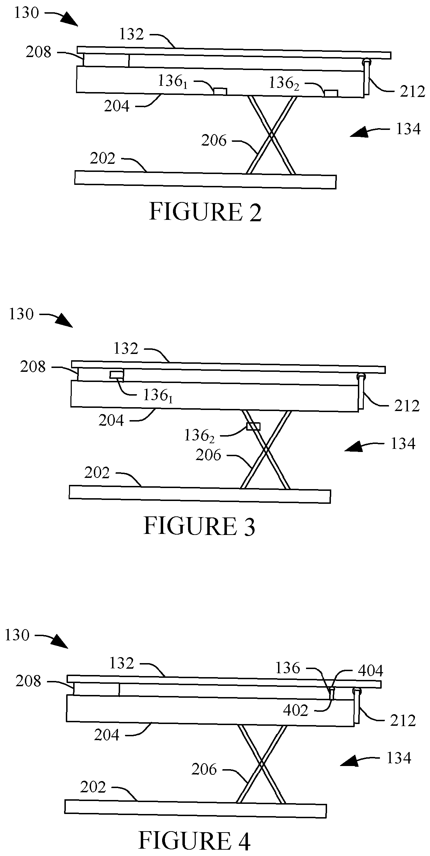

Turning to FIGURES 2, 3 and 4, side views of an example of the table 130 with the at least one sensor 136 is schematically illustrated. In these examples, the base 134 includes a bracket 202 configured to mount to an examination room floor or the like. The base 134 further includes a transporter 204 configured to carry the cradle 132. The base 134 further includes a lift mechanism 206 between the bracket 202 and transporter 204. In the illustrated example, the lift mechanism 206 includes a scissor lift. Other lifts such as telescoping, articulating, etc. are contemplated herein.

A cradle base 208 is slidably attached to the transporter 204 and the cradle 132 is disposed on the cradle base 208. A roller arm 212 supports a free end of the cradle 132 when located outside of the bore 108. As discussed herein, the at least one sensor 136 can be disposed at various different locations of the table 130 and is configured to measure at two or more measurement points of the table 130.

FIGURE 2 schematically illustrates an example where the at least one sensor 136 includes two sensors, a first sensor $136_1$ and a second sensor $136_2$. The first sensor $136_1$ and the second sensor $136_2$ are both disposed on a same component (the transport 204) of the table 130 and are spatially separated to provide two different measurement points. In another instance, the first sensor $136_1$ and the second sensor $136_2$ are both disposed on the bracket 202, both disposed on the lift mechanism 206, both disposed on the cradle base 208 and/or both disposed on the cradle 132, and spatially separated.

FIGURE 3 schematically illustrates an example in which the first sensor $136_1$ and the second sensor $136_2$ are disposed on a different component of the table 130. In this example, the first sensor $136_1$ is disposed on the cradle base 208 and the second sensor $136_2$ is disposed on the lift mechanism 206. Other combinations include, but are not limited to, one sensor both disposed on the bracket 202 and another sensor both disposed on the cradle 132, one sensor both disposed on the bracket 202 and another sensor both disposed on the cradle 132, etc.

FIGURE 4 schematically illustrates an example where the at least one sensor 136 includes a single sensor 136 fixedly attached at one end 402 to the transporter 204 of the table 130, with a free moveable end 404 in physical contact with the cradle 132. An example of a suitable sensor includes a displacement sensor with roller. In this example, the two measurement points correspond to two different horizontal positions of the cradle 132 with respect to the at least one sensor 136. For this, a first measurement is recorded with the cradle 132 at a first location, the cradle 132 is moved to a different location, and a second measurement is recorded with the cradle 132 at the different location.

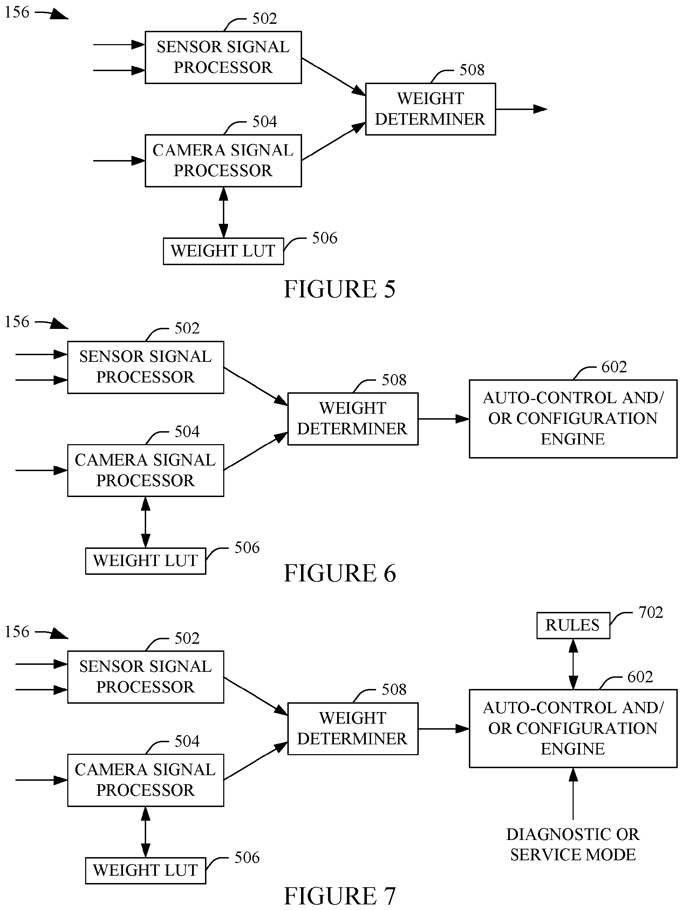

Moving to FIGURES 5, 6 and 7, examples of the subject support module 156 are schematically illustrated.

In FIGURE 5, the subject support module 156 includes a sensor signal processor 502. The sensor signal processor 502 receives, as input, the measurements obtained at the two or more measurement points. Where the measurements include analog data, the sensor signal processor 502 and/or other component samples the analog signals at a predetermined sampling rate and converts them into digital signals. Where the measurements include digital data, the sensor signal processor 502 receives a stream of digital data for one measurement point and another stream of digital data for the other measurement point. In some instances, the sensor signal processor 502 and/or other component conditions the signals, e.g., amplifies, filter, etc. the signals.

The sensor signal processor 502 is configured to determine a weight of the load on the table based on the received signals. By way of non-limiting example, where the at least one sensor 136 includes a strain gauge, the receive signals include strain ($\epsilon$) measurements. The sensor signal processor 502 can determine both weight and position of the load through solving a system of at least two equations with two unknowns. In one instance, the two equations are equations that represent moments around the first and second strain gauges. The output signal of the sensor signal processor 502 is indicative of the weight of the load.

By way of example, where a load is at a distance x from one of two strain gauge sensors 136, the moments around the gauges can be written as shown in EQUATION 1 and EQUATION 2:

$$\text{EQUATION 1:}$$

$$Fx = E\epsilon_1 AL_1,$$

where F represents force (i.e., weight), E represents Young's Modulus, $\epsilon_1$ represents a strain reading of the strain gauge, A represents a cross-sectional area and $L_1$ represents a first distance from the strain gauge to x, and

$$\text{EQUATION 2:}$$

$$F \cdot (L-x) = E\epsilon_2 AL_2,$$

where L represents a distance between the two strain gauges, $\epsilon_2$ represents a strain reading of the other strain gauge, and $L_2$ represents a second distance from the other strain gauge to x. To solve for weight and position, the two equations can be solved simultaneously using algebraic and/or other approaches.

The subject support module 156 further includes a camera signal processor 504. The camera signal processor 504 receives, as input, the signal from the camera 148. In one instance, the camera signal processor 504 includes a classifier configured to identify and classify the individual content on the table 130. For example, in one instance the classifier is configured to differentiate between a subject and an object, different objects, etc. For example, if the table 130 is carrying a subject with a blanket, a brace, a collar, etc., an ECG monitor, a Non-Invasive Blood Pressure (NIBP) device, a container of intravenous fluid, etc., in one instance the classifier identifies and classifies the subject, the blanket, the brace, the collar, the ECG monitor, the NIBP device, the bag of saline, etc., respectively as a subject, a blanket, a brace, a collar, an ECG monitor, an NIBP device, a container of intravenous fluid, etc.

The subject support module 156 further includes a weight lookup table (LUT) 506 or the like. The weight LUT 506 includes predetermined mappings that maps objects to estimated weights. In one instance, the mappings map a category of an object to an estimated weight such as a mean, median, etc. weight of members of the category. For example, ECG monitors are manufactured by different companies. The different ECG monitors, including different models manufactured by the same company, do not all have the exact same weight. In such an instance, a value like a mean value for a population of different ECG can be used as the estimated weight for an identified ECG monitor.

In another instance, a particular category may be delineated into a plurality of sub-categories such as small, medium, large, etc. In such an instance, each of the sub-categories would map to a different estimated weight. With continuance with the above example, each ECG monitor would be classified as a small, medium, large, etc. and map to a corresponding weight. In another instance, the classification is configured to identify and classify a particular manufacturer and/or a model, and the weight LUT 506 includes a weight for a plurality of different manufacturers and/or models.

The output signal of the sensor signal processor 502 is indicative of the weight of the objects. In one instance, the output is a single value representative of the aggregate weight of the identified objects. For this, the sensor signal processor 502 determines the weight of all the identified objects concurrently or sums the individual weights of the identified objects. In this instance, the output may or may not include an identification of each accessory. In another instance, the output includes individual weight values, each corresponding to a different object. In this instance, the output may also include an identification of each object, mapped to its estimated weight.

The weight determiner 508 based on the load and accessory weights, determines the weight of the subject, e.g., by subtracting the weight of the objects from the weight of the load. In one instance, the weight determiner 508 presents the weight of the subject, e.g., visually via a display monitor, audibly via a speaker, etc. In one instance, the weight determiner 508 compares the subject and/or object weight with a predetermined weight limit for the table 130. In such an instance, the weight determiner 508 at least presents a notification when the determined weight exceeds the predetermined weight limit. The weight determiner 508 can further provide the weight to a remote resource such as an EMR for the subject from the remote resources 158.

FIGURE 6 schematically illustrates a variation of the subject support module 156 of FIGURE 5 that further includes an auto-control and/or configuration engine 602. As discussed herein, certain scan protocols and/or certain scan parameters are selected based on a weight of a subject. In this example, the auto-control and/or configuration engine 602, based on the determined weight of the subject by the weight determiner 508, automatically selects a protocol (e.g., infant, child, etc.) and/or auto-populates parameters of a protocol (e.g., kVp, mAs, etc.). The user can confirm, change, edit, reject, accept, any or all of the auto-selected protocols and/or parameters.

FIGURE 7 schematically illustrates a variation of the subject support module 156 of FIGURES 5 and 6 that further includes a set of rules 702 that determine actions for the auto-control and/or configuration engine 602 that are based on a mode of operation, including a diagnostic mode and a service mode. A mode of operation may automatically be determined, e.g., based on the log-in information of the user, which may automatically trigger a diagnostic mode or service mode signal to invoke the auto-configuration engine 602. In another instance, the user identifies the mode of operation via an input. In either mode, a rule from the set of rules 702 invokes the auto-control and/or configuration engine 602 automatically performs certain actions that, respectively, facilitate scanning a subject and service.

In general, diagnostic mode is configured for scanning subjects for medical reasons. A diagnostic mode scan may include assisting and/or positioning a subject on the table 130, executing a scan, and unloading the subject from the table 130. In this example, the auto-control and/or configuration engine 602 assists with setting up scan (e.g., protocol selection, parameter selection, etc.), ensuring safe conditions (e.g., weight limit, not subject movement), etc. Scans tend to be relatively shorter (e.g., minutes), and the operator has visual access to the subject through a window or like.

In general, service mode is configured for installing, configuring, updating, troubleshooting, etc. the imaging system 102. For service mode, a phantom and/or other service accessory may be placed on the table 130, and not subject. A service procedure could be relatively longer (e.g., over an hour, etc.), and visual access to the table 130 may not be readily available and/or feasible. In this example, the auto-control and/or configuration engine 602 provides a notification indicating whether nothing, a service accessory, or an object (e.g. a subject) that should not be on the support is on the support.

Initially referring to diagnostic mode, a rule from the set of rules 702 may invoke the auto-control and/or configuration engine 602 to automatically select a protocol and/or auto-populates parameters of a protocol, as discussed in connection with FIGURE 5.

Another rule, e.g., for an overweight condition, may invoke the auto-control and/or configuration engine 602 to issue a signal which prevents initiation of the imaging scan or pause an imaging scan by preventing X-ray radiation from being generated and/or entering the bore 108 (e.g., turn X-ray generation off, move an X-ray radiation opaque filter in front of the tube window, etc.), the cradle 132 from moving horizontally into the bore 108, the base 134 from moving vertically, selection of a protocol and/or parameters, etc. This rule or another rule may further invoke the auto-control and/or configuration engine 602 to move and/or park the table 130 at a predetermined safe location. The auto-control and/or configuration engine 602 would also provide a visual, audible, etc. notification as discussed above.

Another rule, e.g., for after loading a subject and before unloading the subject, identifies, from the determined weight, a change in weight that is indicative of subject movement. In such an instance, the auto-control and/or configuration engine 602 at least presents a notification to the user. In some instances, the auto-control and/or configuration engine 602 automatically, via a speaker in the examiner room and/or otherwise, provides a message to the subject informing the subject to remain still. In some instances, the change in weight is indicative of a subject removing themselves from the table 130. In this instance, the auto-control and/or configuration engine 602 can provide messages to the user and/or the subject, and/or control certain features of the imaging system 102, such as prevent X-ray radiation from entering the bore 108 (e.g., turn X-ray generation off, move an X-ray radiation opaque filter in front of the tube window, etc.). That is, the auto-control and/or configuration engine 602 can prevent initiation of the imaging scan or pause an imaging scan.

Turing to service mode, a rule from the set of rules 702 may invoke the auto-control and/or configuration engine 602 to visually display a graphic on a display of the output device 146 of the operating console 140 that indicates a current state. In one instance, the graphic indicates whether nothing is on the table 130, a service accessory such as a phantom is on the table 130, something that should not be on the table 130 is on the table 130. For example, the sensor signal and/or the camera signal may indicate that a phantom is on the table 130, and this information can be communicated to the service technician through the graphic displayed on a monitor and/or otherwise.

In another example, if another person were to place something on the table 130 or remove a service accessory off of the table 130, or a subject were to get on the table 130, the auto-control and/or configuration engine 602 would detect the change in weight, and, along with the camera signal, set the state of the graphic to indicate the current state. For example, where the service technician loaded a phantom for certain testing, and the phantom is removed, the graphic would indicate that there is no longer a phantom on the table 130. In this instance, the auto-control and/or configuration engine 602 may present the weight and/or the camera signal on a display of the operating console 140 for observance by the service technician. The service technician can reinstall the phantom and resume the service procedure.

In another example, where the auto-control and/or configuration engine 602 determines, based on the weight and/or the camera signal, that something other than a phantom is on the table 130. In this instance, the auto-control and/or configuration engine 602 sets the state of the graphic to indicate something is on the table 130 and control the imaging system 102 to prevent X-ray radiation from entering the bore 108 (e.g., turn X-ray generation off, move an X-ray radiation opaque filter in front of the tube window, etc.). That is, the auto-control and/or configuration engine 602 can prevent initiation of the imaging scan or pause an imaging scan. In this instance, the auto-control and/or configuration engine 602 may present the weight and/or the camera signal on a display of the operating console 140 for observance by the service technician. Once the item is removed or the service technician overrides the warning, the service technician can resume the service procedure.

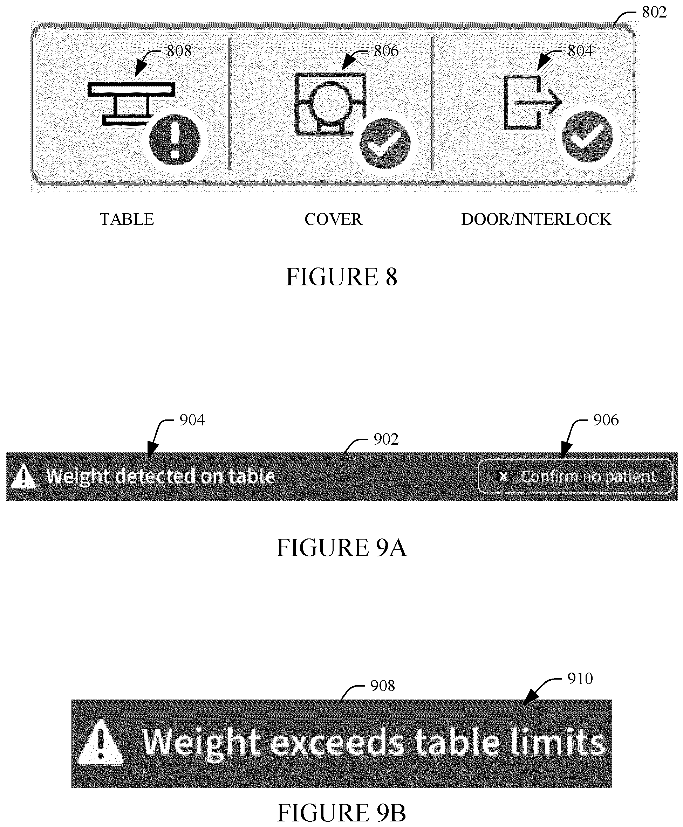

FIGURES 8, 9A and 9B depict example state information graphics that can be utilized in the diagnostic and/or service modes discussed herein. In FIGURE 8, a graphic 802 includes multiple icons, including a door/-interlock icon 804, a cover icon 806, and a table icon (which corresponds to the table 130) 808. The door/-interlock icon 804 indicates whether the door/interlock is open or closed. In FIGURE 8, the "check mark" graphic over a portion of the door/interlock icon 804 indicates the door/interlock is closed. The cover icon 806 indicates whether the gantry cover is open or closed. In FIGURE 8, the "check mark" graphic over a portion of the cover icon 806 indicates the cover is closed.

The table icon 808, for diagnostic mode, indicates whether or not there is a potential issue with the table 130. Where a subject is loaded, their weight is within maximum weight specifications, and the subject is lying in accordance with instructions from the operator, a "check mark" graphic (similar to the "check mark" graphics over icons 804 and 806) would be over a portion of the table icon 808 to indicate there is no potential issue. In the illustrated example, a warning mark is over a portion of the table icon 808. Depending on the situation, this could mean one or more of an overweight limit, weight over a threshold value has been added, weight over a threshold value has been removed, and/or other condition that requires the attention of the user.

The table icon 808, for service mode, likewise indicates whether or not there is a potential issue with the table 130. Where an accessory is installed for a service procedure, a "check mark" graphic (similar to the "check mark" graphics over icons 804 and 806) would be over a portion of the table icon 808 to indicate there is no potential issue. Where an accessory is not installed for a service procedure, a "check mark" graphic (similar to the "check mark" graphics over icons 804 and 806) would be over a portion of the table icon 808 to indicate there is no potential issue. In the illustrated example, a warning mark is over a portion of the table icon 808. Depending on the situation, this could mean that the measured weight is outside of a weight range (below or above) for the installed service accessory or no accessory.

FIGURES 9A illustrates an example of a pop-up banner 902 that is presented on a display monitor such as the operator console 140 when the subject support module 156 detects weight on the table 130. The pop-up banner alerts the operator or service technician via a message 904 and is removed from the display after the operator or service technician confirms that there is no patient on (or no longer on) the table 130, e.g., by activating (e.g., "clicking" on) a "Confirm no patient" active button 906 of the pop-up banner 902.

FIGURE 9B illustrates an example of a pop-up banner 908 that is presented on a display monitor such as the operator console 140 when the subject support module 156 detects that weight on the table 130 exceeds a weight limit of the table 130. The pop-up banner alerts the operator or service technician via a message 910 and, in one instance, continues to be presented until the subject support module 156 detects the weight on the table 130 satisfies the weight limit of the table 130. The weight may be a weight of a subject, a weight of one or more accessories, or a weight of a combination of a subject and one or more accessories. FIGURES 8, 9A and 9B provide non-limiting examples, and other notices, messages, warnings, etc. are contemplated herein.

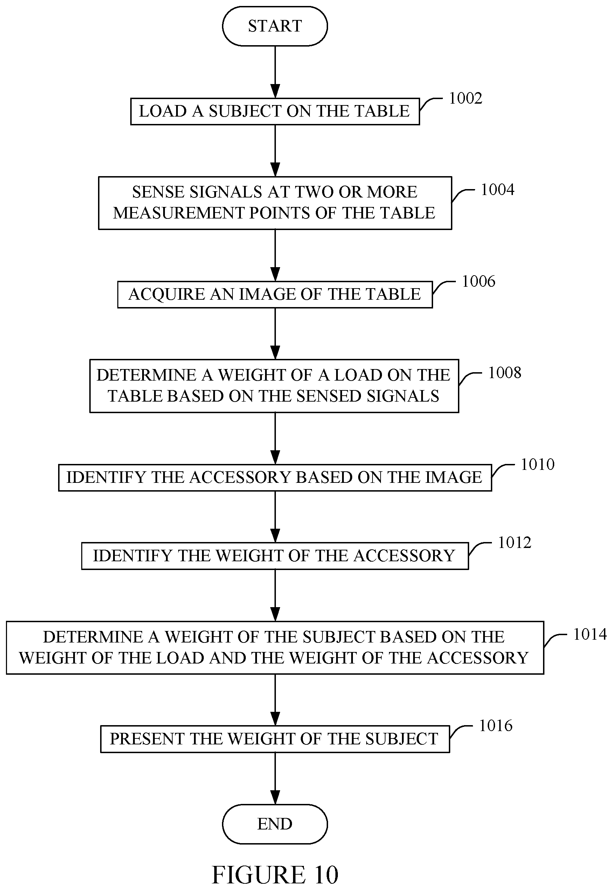

FIGURE 10 illustrates a non-limiting example of a flow chart for a computer-implemented method for measuring a weight of a subject being scanned, in accordance with an aspect herein. It is to be appreciated that the ordering of the acts in the method is not limiting. As such, other orderings are contemplated herein. In addition, one or more acts may be omitted, and/or one or more additional acts may be included.

At 1002, a subject (along with at least one accessory) is loaded on the table 130 for scanning, as described herein and/or otherwise. As described herein, the at least one accessory can include an accessory carried by the subject such as a blanket, a brace, a collar, etc., and/or a separate item such as an ECG monitor, an NIBP monitor, etc. At 1004, signals at two or more different measurement points of the table 130 are sensed, as described herein and/or otherwise. At 1006, an image of content carried by cradle 132 of the table 130 is acquired, as described herein and/or otherwise.

At 1008, a weight of a load (the subject and the accessory) carried by the table 130 is determined based on the sensed signals, as described herein and/or otherwise. At 1010, the accessory is identified based on the acquired image, as described herein and/or otherwise. At 1012, a weight of the accessory is identified, as described herein and/or otherwise. At 1014, a weight of the subject is determined based on the weight of the load and the weight of the accessory, as described herein and/or otherwise. At 1016, the weight of the subject is presented, as described herein and/or otherwise.

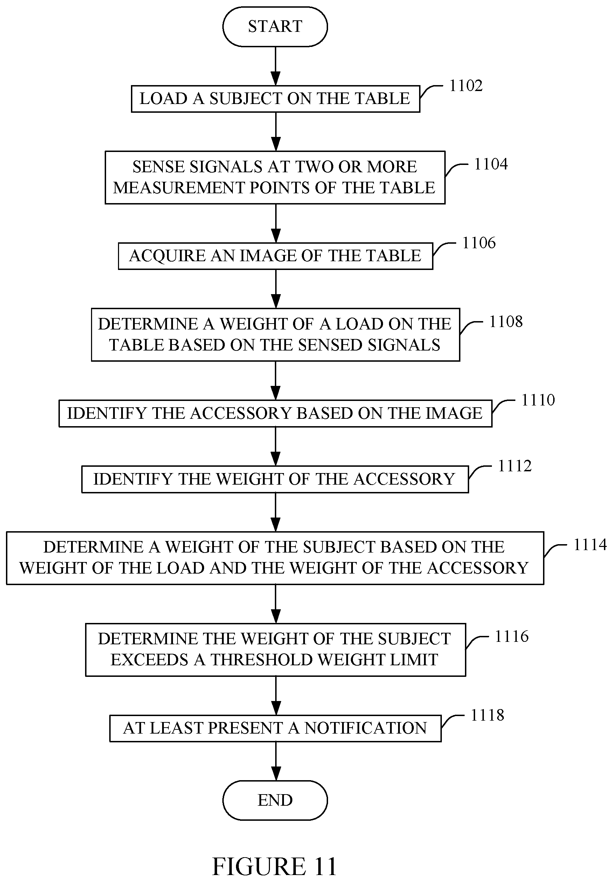

FIGURE 11 illustrates a non-limiting example of a flow chart for a computer-implemented method for sensing whether a subject exceeds the weight limit of the table 130, in accordance with an aspect herein. It is to be appreciated that the ordering of the acts in the method is not limiting. As such, other orderings are contemplated herein. In addition, one or more acts may be omitted, and/or one or more additional acts may be included.

At 1102, a subject (along with at least one accessory) is loaded on the table 130 for scanning, as described herein and/or otherwise. At 1104, signals at two or more different measurement points of the table 130 are sensed, as described herein and/or otherwise. At 1106, an image of content carried by cradle 132 of the table 130 is acquired, as described herein and/or otherwise. At 1108, a weight of a load (the subject and the accessory) carried by the table 130 is determined based on the sensed signals, as described herein and/or otherwise.

At 1110, the accessory is identified based on the acquired image, as described herein and/or otherwise. At 1112, a weight of the accessory is identified, as described herein and/or otherwise. At 1114, a weight of the subject is determined based on the weight of the load and the weight of the accessory, as described herein and/or otherwise. At 1116, the weight of the subject is determined to exceed a threshold weight limit of the table 130, as described herein and/or otherwise. At 1118, a notification is presented that indicates that the weight of the subject exceeds the threshold weight limit, as described herein and/or otherwise.

Optionally, the imaging system 102 is automatically controlled so that X-ray radiation cannot enter the examiner region 108, as described herein and/or otherwise. Optionally, the table 130 is automatically controlled to prevent horizontal and/or vertical movement, as described herein and/or otherwise. Optionally, the imaging system 102 is automatically controlled to inhibit protocol selection and/or scan parameter entry, as described herein and/or otherwise.

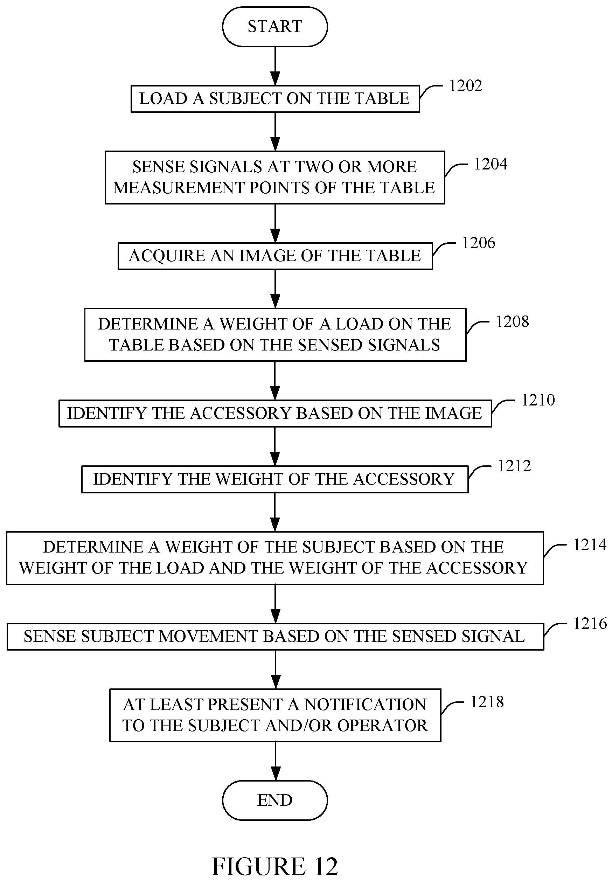

FIGURE 12 illustrates a non-limiting example of a flow chart for a computer-implemented method for sensing subject movement on the table 130 during scanning, in accordance with an aspect herein. It is to be appreciated that the ordering of the acts in the method is not limiting. As such, other orderings are contemplated herein. In addition, one or more acts may be omitted, and/or one or more additional acts may be included.

At 1202, a subject (along with at least one accessory) is loaded on the table 130 for scanning, as described herein and/or otherwise. At 1204, signals at two or more different measurement points of the table 130 are sensed, as described herein and/or otherwise. At 1206, an image of a top of the cradle 132 of the table 130 is acquired, as described herein and/or otherwise. At 1208, a weight of a load carried by the table 130 is determined based on the sensed signals, as described herein and/or otherwise.

At 1210, the accessory based on the acquired image is identified, as described herein and/or otherwise. At 1212, a weight of the accessory is identified, as described herein and/or otherwise. At 1214, a weight of the subject is determined based on the weight of the load and the weight of the accessory, as described herein and/or otherwise. At 1216, subject movement is sensed based on the signals, as described herein and/or otherwise. At 1218, a notification is presented to the subject and/or operator indicating the movement, as described herein and/or otherwise. In one instance, an auto-voice feature audibly informs via a speaker the subject to remain still for the scan, as described herein and/or otherwise.

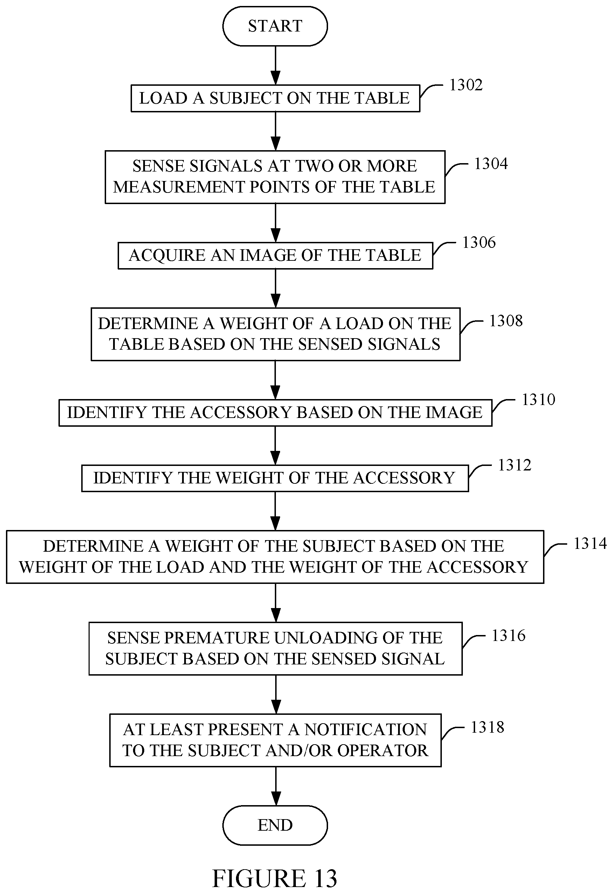

FIGURE 13 illustrates a non-limiting example of a flow chart for a computer-implemented method for sensing premature unloading of a subject from the table 130 by subject movement on the table 130 during scanning, in accordance with an aspect herein. It is to be appreciated that the ordering of the acts in the method is not limiting. As such, other orderings are contemplated herein. In addition, one or more acts may be omitted, and/or one or more additional acts may be included.

At 1302, a subject (along with at least one accessory) is loaded on the table 130 for scanning, as described herein and/or otherwise. At 1304, signals at two or more different measurement points of the table 130 are sensed, as described herein and/or otherwise. At 1306, an image of a top of the cradle 132 of the table 130 is acquired, as described herein and/or otherwise. At 1308, a weight of a load carried by the table 130 is determined based on the sensed signals, as described herein and/or otherwise.

At 1310, the accessory based on the acquired image is identified, as described herein and/or otherwise. At 1312, a weight of the accessory is identified, as described herein and/or otherwise. At 1314, a weight of the subject is determined based on the weight of the load and the weight of the accessory, as described herein and/or otherwise. At 1316, premature unloading of the subject is sensed based on the signals, as described herein and/or otherwise. At 1318, a notification is presented to the operator indicating the premature unloading, as described herein and/or otherwise. Optionally, certain features such as X-ray radiation generation, transmission, etc. is disabled, prevented, and/or inhibited.

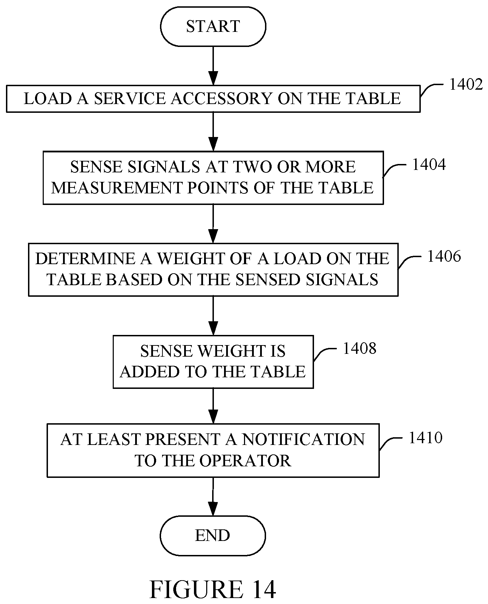

FIGURE 14 illustrates a non-limiting example of a flow chart for a computer-implemented method for sensing a subject on the table 130, in accordance with an aspect herein. It is to be appreciated that the ordering of the acts in the method is not limiting. As such, other orderings are contemplated herein. In addition, one or more acts may be omitted, and/or one or more additional acts may be included.

At 1402, a service accessory is loaded on the table 130 for service, as described herein and/or otherwise. In another instance, no accessory is loaded on the table 130 for service, as described herein and/or otherwise. At 1404, signals are sensed at two or more different measurement points of the table 130, as described herein and/or otherwise. At 1406, a weight of a load (or no load) carried by the table 130 is determined based on the sensed signals, as described herein and/or otherwise. At 1408, a change in the weight is identified based on the sensed signals, as described herein and/or otherwise. At 1410, a notification is presented to the user indicating weight was added to the table 130, as described herein and/or otherwise.

In one instance, this includes presenting a graphic representing a state of the imaging system 102 in which the graphic indicates that the service accessory or nothing is loaded on the table, and then updating the graphic to indicate that something was added to the table. Optionally, certain features are disabled, prevented, and/or inhibited as described herein. In another instance, the graphic is presented only after the added weight was detected. In another instance, the signal from the camera 148 is processed to identify the added object. In one instance, the user either confirms the object is not a subject or, where the object is a subject, that the object is no longer on the table.

The above can be implemented by way of computer readable instructions, encoded, or embedded on the computer readable storage medium, which, when executed by a computer processor, cause the processor to carry out the described acts or functions. Additionally, or alternatively, at least one of the computer readable instructions is carried out by a signal, carrier wave or other transitory medium, which is not computer readable storage medium.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include such additional elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

The various embodiments and/or components, for example, the modules, or components and controllers therein, also may be implemented as part of one or more computers or processors. The computer or processor may include a computing device, an input device, a display unit and an interface, for example, for accessing the Internet. The computer or processor may include a microprocessor. The microprocessor may be connected to a communication bus. The computer or processor may also include a memory. The memory may include Random Access Memory (RAM) and Read Only Memory (ROM). The computer or processor further may include a storage device, which may be a hard disk drive or a removable storage drive such as a floppy disk drive, optical disk drive, and the like. The storage device may also be other similar means for loading computer programs or other instructions into the computer or processor.

As used herein, the term "computer" or "module" may include any processor-based or microprocessor-based system including systems using microcontrollers, reduced instruction set computers (RISC), application specific integrated circuits (ASICs), logic circuits, and any other circuit or processor capable of executing the functions described herein. The above examples are exemplary only, and are thus not intended to limit in any way the definition and/or meaning of the term "computer". The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present disclosure. That is, features of the described embodiments can be combined with any appropriate aspect described above and optional features of any one aspect can be combined with any other appropriate aspects. Similarly, features set forth in dependent claims can be combined with non-mutually exclusive features of other dependent claims, particularly where the dependent claims depend on the same independent claim. Single claim dependencies may have been used as practice in some jurisdictions that require them, but this should not be taken to mean that the features in the dependent claims are mutually exclusive.

## Claims

1. A medical imaging system, comprising:

   a gantry including a bore extending through the gantry;
   a table configured to support a subject moving through the bore;
   at least one sensor disposed on the table and configured to sense a total weight of a load on the table at two or more different measurement points;
   a camera configured to generate an image of the table including the subject; and
   a processor configured to determine a weight of the subject on the table based on at least one signal from the at least one sensor and a second signal from the camera.

2. The medical imaging system of claim 1, wherein the total weight of the load includes the weight of the subject and a weight of at least one accessory, and the processor is further configured to determine the weight of the at least one accessory based on the second signal from the camera, and determine the weight of the subject based on a difference between the total weight of the load and the weight of the at least one accessory.

3. The medical imaging system of claim 2, wherein the processor is further configured to identify the at least one accessory based on the second signal from the camera, and determine the weight of the at least one accessory based on the identification of the at least one accessory.

4. The medical imaging system of claim 1, wherein the at least one force sensor includes:

   a first sensor disposed at a first location of the table and configured to sense at a first measurement point; and
   a second sensor disposed at a second location of the table and configured to sense at a second measurement point.

5. The medical imaging system of claim 1, wherein the table includes:

   a base; and
   a cradle moveably disposed on the base, and wherein the at least one sensor includes:
   a single sensor disposed at a static location of the base and configured to sense at a first measurement point at a first location of the cradle and a second measurement point at a second location of the cradle.

6. The medical imaging system of claim 1, wherein the processor is further configured to at least one of automatically select an imaging protocol for the subject and automatically populate at least one imaging parameter of the imaging protocol based on the weight of the subject.

7. The medical imaging system of claim 1, wherein the processor is further configured to determine whether the weight of the subject exceeds a predetermined weight limit of the table, and, in response to the weight of the subject exceeding the predetermined weight limit, and the processor is further configured to present a notification indicating the weight of the subject exceeds the predetermined weight limit.

8. The medical imaging system of claim 7, wherein the processor, in response to the weight of the subject exceeding the predetermined weight limit, is further configured to prevent initiation of an imaging scan.

9. The medical imaging system of claim 1, wherein the processor is further configured to identify a movement of the subject on the table based on the at least one signal and, in response to identifying the movement, present a notification to the subject to remain still.

10. The medical imaging system of claim 1, wherein the processor is further configured to identify the subject is no longer on the table based on the at least one signal and, in response to identifying the subject is no longer on the table, present a notification indicating the subject is no longer on the table and pause an imaging scan.

11. The medical imaging system of claim 1, wherein the processor is further configured to determine when a subject is on the table based on the weight of the subject and present a notification indicating whether the subject is on the table.

12. A computer-implemented method, comprising:

receiving at least one signal from at least one sensor disposed on a table of a medical imaging system, wherein the at least one signal includes measurements from two or more different measurement points of the table; receiving a second signal from an optical sensor configured to generate an image of the table; and determining a weight of a subject on the table based on the at least one signal and the second signal.

13. The computer-implemented method of claim 12, further comprising, at least one of:

automatically selecting an imaging protocol for the subject based on the weight of the subject; and

automatically populating at least one imaging parameter of the imaging protocol based on the weight of the subject.

14. The computer-implemented method of claim 12, further comprising:

determining the weight of the subject exceeds a predetermined weight limit of the table; and presenting a notification indicating the weight of the subject exceeds the predetermined weight limit.

15. The computer-implemented method of claim 12, further comprising:

determining a movement of the subject based on the at least one signal and the second signal; and presenting a notification to the subject to stay still.

**FIGURE 1**

FIGURE 2

FIGURE 3

FIGURE 4

156 →

502
SENSOR SIGNAL
PROCESSOR

508
WEIGHT
DETERMINER

504
CAMERA SIGNAL
PROCESSOR

506
WEIGHT LUT

FIGURE 5

156 →

502
SENSOR SIGNAL
PROCESSOR

508
WEIGHT
DETERMINER

602
AUTO-CONTROL AND/
OR CONFIGURATION
ENGINE

504
CAMERA SIGNAL
PROCESSOR

506
WEIGHT LUT

FIGURE 6

156 →

702
RULES

502
SENSOR SIGNAL
PROCESSOR

508
WEIGHT
DETERMINER

602
AUTO-CONTROL AND/
OR CONFIGURATION
ENGINE

504
CAMERA SIGNAL
PROCESSOR

506
WEIGHT LUT

DIAGNOSTIC OR
SERVICE MODE

FIGURE 7

TABLE      COVER      DOOR/INTERLOCK

FIGURE 8

FIGURE 9A

FIGURE 9B

START

LOAD A SUBJECT ON THE TABLE — 1002

SENSE SIGNALS AT TWO OR MORE
MEASUREMENT POINTS OF THE TABLE — 1004

ACQUIRE AN IMAGE OF THE TABLE — 1006

DETERMINE A WEIGHT OF A LOAD ON THE
TABLE BASED ON THE SENSED SIGNALS — 1008

IDENTIFY THE ACCESSORY BASED ON THE IMAGE — 1010

IDENTIFY THE WEIGHT OF THE ACCESSORY — 1012

DETERMINE A WEIGHT OF THE SUBJECT BASED ON THE
WEIGHT OF THE LOAD AND THE WEIGHT OF THE ACCESSORY — 1014

PRESENT THE WEIGHT OF THE SUBJECT — 1016

END

FIGURE 10

START

LOAD A SUBJECT ON THE TABLE — 1102

SENSE SIGNALS AT TWO OR MORE
MEASUREMENT POINTS OF THE TABLE — 1104

ACQUIRE AN IMAGE OF THE TABLE — 1106

DETERMINE A WEIGHT OF A LOAD ON THE
TABLE BASED ON THE SENSED SIGNALS — 1108

IDENTIFY THE ACCESSORY BASED ON THE IMAGE — 1110

IDENTIFY THE WEIGHT OF THE ACCESSORY — 1112

DETERMINE A WEIGHT OF THE SUBJECT BASED ON THE
WEIGHT OF THE LOAD AND THE WEIGHT OF THE ACCESSORY — 1114

DETERMINE THE WEIGHT OF THE SUBJECT
EXCEEDS A THRESHOLD WEIGHT LIMIT — 1116

AT LEAST PRESENT A NOTIFICATION — 1118

END

FIGURE 11

```
                    ( START )
                        |
                        v
    +-----------------------------------------+
    | LOAD A SUBJECT ON THE TABLE             |-- 1202
    +-----------------------------------------+
                        |
                        v
    +-----------------------------------------+
    | SENSE SIGNALS AT TWO OR MORE            |-- 1204
    | MEASUREMENT POINTS OF THE TABLE         |
    +-----------------------------------------+
                        |
                        v
    +-----------------------------------------+
    | ACQUIRE AN IMAGE OF THE TABLE           |-- 1206
    +-----------------------------------------+
                        |
                        v
    +-----------------------------------------+
    | DETERMINE A WEIGHT OF A LOAD ON THE     |-- 1208
    | TABLE BASED ON THE SENSED SIGNALS       |
    +-----------------------------------------+
                        |
                        v
    +-----------------------------------------+
    | IDENTIFY THE ACCESSORY BASED ON THE IMAGE|-- 1210
    +-----------------------------------------+
                        |
                        v
    +-----------------------------------------+
    | IDENTIFY THE WEIGHT OF THE ACCESSORY    |-- 1212
    +-----------------------------------------+
                        |
                        v
    +-----------------------------------------+
    | DETERMINE A WEIGHT OF THE SUBJECT BASED ON THE |-- 1214
    | WEIGHT OF THE LOAD AND THE WEIGHT OF THE ACCESSORY|
    +-----------------------------------------+
                        |
                        v
    +-----------------------------------------+
    | SENSE SUBJECT MOVEMENT BASED ON THE SENSED SIGNAL |-- 1216
    +-----------------------------------------+
                        |
                        v
    +-----------------------------------------+
    | AT LEAST PRESENT A NOTIFICATION         |-- 1218
    | TO THE SUBJECT AND/OR OPERATOR          |
    +-----------------------------------------+
                        |
                        v
                    ( END )
```

FIGURE 12

START

LOAD A SUBJECT ON THE TABLE — 1302

SENSE SIGNALS AT TWO OR MORE
MEASUREMENT POINTS OF THE TABLE — 1304

ACQUIRE AN IMAGE OF THE TABLE — 1306

DETERMINE A WEIGHT OF A LOAD ON THE
TABLE BASED ON THE SENSED SIGNALS — 1308

IDENTIFY THE ACCESSORY BASED ON THE IMAGE — 1310

IDENTIFY THE WEIGHT OF THE ACCESSORY — 1312

DETERMINE A WEIGHT OF THE SUBJECT BASED ON THE
WEIGHT OF THE LOAD AND THE WEIGHT OF THE ACCESSORY — 1314

SENSE PREMATURE UNLOADING OF THE
SUBJECT BASED ON THE SENSED SIGNAL — 1316

AT LEAST PRESENT A NOTIFICATION
TO THE SUBJECT AND/OR OPERATOR — 1318

END

FIGURE 13

START

LOAD A SERVICE ACCESSORY ON THE TABLE — 1402

SENSE SIGNALS AT TWO OR MORE
MEASUREMENT POINTS OF THE TABLE — 1404

DETERMINE A WEIGHT OF A LOAD ON THE
TABLE BASED ON THE SENSED SIGNALS — 1406

SENSE WEIGHT IS
ADDED TO THE TABLE — 1408

AT LEAST PRESENT A NOTIFICATION
TO THE OPERATOR — 1410

END

FIGURE 14

Europäisches
Patentamt

European
Patent Office

Office européen
des brevets

## EUROPEAN SEARCH REPORT

**Application Number**

EP 25 20 0437

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 385 419 A1 (CANON MEDICAL SYSTEMS CORP [JP]) 19 June 2024 (2024-06-19) | 1,4,5,8, 10,12 | INV. A61B6/00 |
| Y | * paragraph [0044] - paragraph [0149]; figures 5-8 * | 6,7,9, 11,13-15 | A61B6/03 A61B6/04 |
| A |  | 2,3 | A61B5/055 |
|  | ----- |  |  |
| Y | US 2009/252300 A1 (SCHWARTZ MATTHEW RICHARD [US] ET AL) 8 October 2009 (2009-10-08) * paragraph [0019] - paragraph [0022] * | 6,7,13, 14 |  |
|  | ----- |  |  |
| Y | EP 3 673 806 A2 (GEN ELECTRIC [US]) 1 July 2020 (2020-07-01) * paragraph [0048] - paragraph [0050] * | 9,15 |  |
|  | ----- |  |  |
| Y | US 2022/015710 A1 (LEWIS CHELSEY A [US] ET AL) 20 January 2022 (2022-01-20) * paragraph [0063] * | 11 |  |
|  | ----- |  |  |

**TECHNICAL FIELDS SEARCHED (IPC)**

A61B

The present search report has been drawn up for all claims

| Place of search | Date of completion of the search | Examiner |
|---|---|---|
| Munich | 5 February 2026 | Hooper, Martin |

EPO FORM 1503 03.82 (P04C01)

## ANNEX TO THE EUROPEAN SEARCH REPORT
## ON EUROPEAN PATENT APPLICATION NO.

EP 25 20 0437

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2026

| Patent document cited in search report | | Publication date | Patent family member(s) | | Publication date |
|---|---|---|---|---|---|
| EP 4385419 | A1 | 19-06-2024 | EP | 4385419 A1 | 19-06-2024 |
| | | | US | 2024188849 A1 | 13-06-2024 |
| US 2009252300 | A1 | 08-10-2009 | CN | 101548888 A | 07-10-2009 |
| | | | JP | 2009247895 A | 29-10-2009 |
| | | | US | 2009252300 A1 | 08-10-2009 |
| EP 3673806 | A2 | 01-07-2020 | CN | 111374675 A | 07-07-2020 |
| | | | EP | 3673806 A2 | 01-07-2020 |
| | | | JP | 7242514 B2 | 20-03-2023 |
| | | | JP | 2020121104 A | 13-08-2020 |
| | | | US | 2020205748 A1 | 02-07-2020 |
| US 2022015710 | A1 | 20-01-2022 | CN | 113940691 A | 18-01-2022 |
| | | | US | 2022015710 A1 | 20-01-2022 |

EPO FORM P0459

For more details about this annex : see Official Journal of the European Patent Office, No. 12/82